(19) Europäisches Patentamt
European Patent Office
Office européen des brevets

(11) Veröffentlichungsnummer: **0 169 405**
**A2**

# (12) EUROPÄISCHE PATENTANMELDUNG

(21) Anmeldenummer: **85108005.1**

(22) Anmeldetag: **28.06.85**

(51) Int. Cl.[4]: **A 61 K 31/505**
A 61 K 31/55, A 61 K 31/535
A 61 K 31/54
//C07D487/04

(30) Priorität: **19.07.84 DE 3426615**

(43) Veröffentlichungstag der Anmeldung:
**29.01.86 Patentblatt 86/5**

(84) Benannte Vertragsstaaten:
**AT BE CH DE FR GB IT LI LU NL SE**

(71) Anmelder: **BOEHRINGER INGELHEIM KG**

**D-6507 Ingelheim am Rhein(DE)**

(84) Benannte Vertragsstaaten:
**BE CH DE FR IT LI LU NL SE AT**

(71) Anmelder: **Boehringer Ingelheim International G.m.b.H**

**D-6507 Ingelheim am Rhein(DE)**

(84) Benannte Vertragsstaaten:
**GB**

(72) Erfinder: **Hinzen, Dieter, Prof. Dr.**
**Konrad-Adenauer-Strasse 26**
**D-6501 Zornheim(DE)**

(72) Erfinder: **Roch, Josef, Dr.**
**Stecherweg 19**
**D-7950 Biberach an der Riss(DE)**

(54) **Neue Verwendung von substituierten Pyrimido-5,4-dpyrimidinen.**

(57) Die aus der DE-A 1 23 oo 661 bekannten Pyrimido[5,4-d]-pyrimidine eignen sich zur Behandlung der zerebralen Insuffizienz. Die Verbindungen zeigen bei gegenüber Vergleichssubstanzen niedrigerer Dosierbarkeit lange Wirkungsdauer sowie neuartige Wirkqualitäten.

EP 0 169 405 A2

Croydon Printing Company Ltd.

Die Erfindung betrifft eine neue Verwendung bekannter substituierter Pyrimido/5,4-d/pyrimidine.

In der DE-A 23 00 661 sind substituierte Pyrimido/5,4-d/pyrimidine der Formel

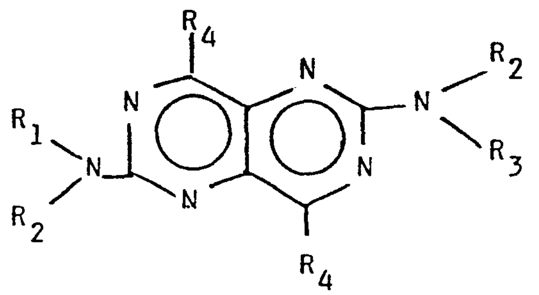

(I)

beschrieben worden, die neben einer Hemmwirkung auf die Thrombozytenaggregation eine durchblutungsfördernde, coronardilatorische und/oder blutdrucksteigernde Wirkung zeigen und außerdem den Sauerstofftransport des Blutes günstig beeinflussen.

In der Formel I bedeutet

$R_1$ einen $C_1$-$C_3$-Alkoxy-($C_2$-$C_3$-alkyl)rest,

$R_2$ einen $C_2$-$C_6$-Hydroxyalkylrest oder einen $C_3$-Dihydroxyalkylrest,

$R_3$ einen $C_1$-$C_3$-Alkoxy-($C_2$-$C_3$-alkyl) rest oder einen $C_2$-$C_3$-Hydroxyalkylrest,

$R_4$ einen Dialkylaminorest, wobei jeder Alkylrest 1 bis 6 C-Atome enthalten kann, die Diallyl- oder Di-(methoxyethyl)-aminogruppe oder einen gegebenenfalls durch eine Methylgruppe substituierten Pyrrolidino-, Piperidino-, Hexamethylenamino-, 1', 2', 5', 6'-Tetrahydropyridino-, Morpholino-, Thiomorpholino- oder 1-Oxidothiomorpholinoring.

Die Verbindungen können als freie Basen oder als physiologisch verträgliche Säureadditionssalze mit anorganischen oder organischen Säuren vorliegen.

Es wurde nun in tierexperimentellen Untersuchungen gefunden, daß die Verbindungen überraschenderweise besonders zur Beeinflussung eingeschränkter zerebraler Leistungsfähigkeit geeignet sind.
Die folgenden Ergebnisse stützen diese Aussage:

1. Durch Gabe des muskarinen cholinergen Antagonisten Scopolamin wird eine Störung des Kurzzeitgedächtnisses bzw. des Übergangs von Inhalten des Kurzzeit- in das sogenannte Langzeitgedächnis erzeugt. Diese Einschränkung wird durch die neuen Verbindungen aufgehoben. Die niedrigste wirksame Dosis der Verbindungen der Formel I beträgt in diesem Prüfverfahren 5 - 10 mg/kg p.o.; die Verbindungen sind damit Vergleichssubstanzen, z.B. Piracetam (wirksam ab 150 mg/kg p.o.) und Aniracetam (wirksam ab 50 mg/kg p.o.) überlegen.

2. Die Habituation von Ratten in einer neuen Umgebung wird in weitem Dosisbereich gleichbleibend stark verbessert. Dies kann als Steigerung der Erinnerungsfähigkeit der Tiere durch die neuen Verbindungen gedeutet werden. Auch hier zeichnen sich diese Verbindungen gegenüber den genannten Vergleichssubstanzen durch niedrigere Dosierbarkeit (wirksam ab 1,25 mg/kg p.o. gegenüber 30 - 150 mg/kg p.o.) und längere Wirkungsdauer vorteilhaft aus.

3. EEG-Untersuchungen an der Katze weisen eine vigilanzsteigernde Wirkung schon ab 1 mg/kg i.p. nach. Die Vigilanzsteigerung zeigt sich in einer Zunahme des kritischen Wachzustandes der Tiere bei Verminderung der Gesamtschlafzeit.

   Bei Kaninchen wird eine zentral nervöse Aktivierung in Dosisbereichen ab o,3 mg/kg i.v. gefördert. Die geprüften Vergleichssubstanzen weisen keine entsprechenden vigilanzfördernden Wirkungen auf.

4. Die lokale Durchblutung in der Großhirnrinde wurde bei Katzen, die mit Pentobarbital anaesthetisiert waren, mit der Thermodilutionsmethode bestimmt (Golenhofen et al., Thieme Verlag, Stuttgart (1963)). Es wurde ein erwärmtes und ein nichterwärmtes Thermoelementenpaar benutzt, wobei der Abstand zwischen den Paaren 4 mm betrug. Änderungen der Thermodifferenzen dienten als Maß für die Durchblutung.

5. Für die Verbindung (B) auf Seite 5 beträgt die i.v. $LD_{50}$ an der Maus 133 mg/kg, die p.o. $LD_{50} > 3$ g/kg.

Aufgrund der neuen tierexperimentellen Befunde werden die Verbindungen der Formel I für die Anwendung im Rahmen der zerebralen Insuffizienz bzw. der posttraumatischen und alkoholischen Hirnschädigung in der Humanmdeizin vorgeschlagen. Im Zusammenhang mit dieser Anwendung sind die oben erwähnten vorbeschriebenen Wirkungen als vorteilhafte Nebenwirkungen zu betrachten.

Im Rahmen der obigen Definitionen können die Substituenten $R_1$ bis $R_3$ gleich oder verschieden sein. Bedeutet $R_4$ eine Dialkylaminogruppe, so können die beiden Alkylgruppen gleich oder verschieden sein. Soweit die Substituenten $R_1$ bis $R_4$ hydroxy- oder alkoxysubstituierte Alkylgrüppen darstellen bzw. enthalten, sind die Hydroxy- oder Alkoxygruppen nicht an dasjenige C-Atom gebunden, das mit dem Stickstoffatom verknüpft ist.

Dementsprechend stehen $R_1$ und $R_3$ z.B. für $CH_2-CH_2-OCH_3$, $CH_2-CH_2-OC_2H_5$, $CH_2-CH_2-CH_2-OCH_3$,
$R_2$ für $CH_2-CH_2-OH$, $(CH_2)_3-OH$, $(CH_2)_4-OH$, $(CH_2)_6-OH$, $CH_2-CH(OH)-CH_3$, $CH_2-C(CH_3)_2OH$,
$R_4$ steht beispielsweise für $N(C_2H_5)_2$, $N(C_3H_7)_2$, $N(C_4H_9)_2$, $N(C_2H_5)(C_3H_7)$, $N(C_2H_5)(C_4H_9)$, $N(C_2H_4OCH_3)_2$, $N(C_2H_4OC_2H_5)_2$.
Soweit $R_4$ einen stickstoffhaltigen heterocyclischen Rest bedeutet, ist dieser über Stickstoff mit dem Pyrimido[5,4-d]pyrimidin-System verknüpft.

Als besonders wirksam haben sich die Verbindungen der Formeln

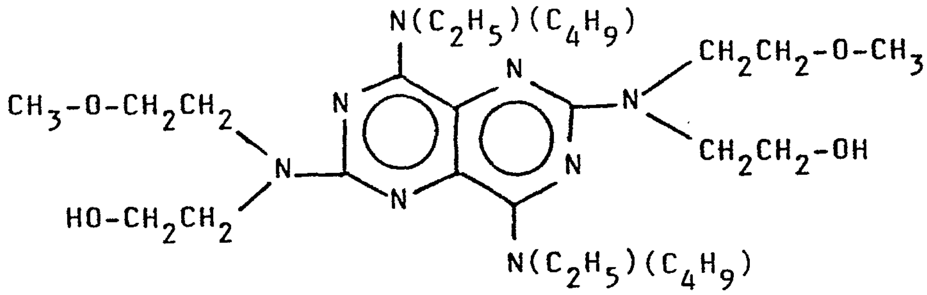

(A)

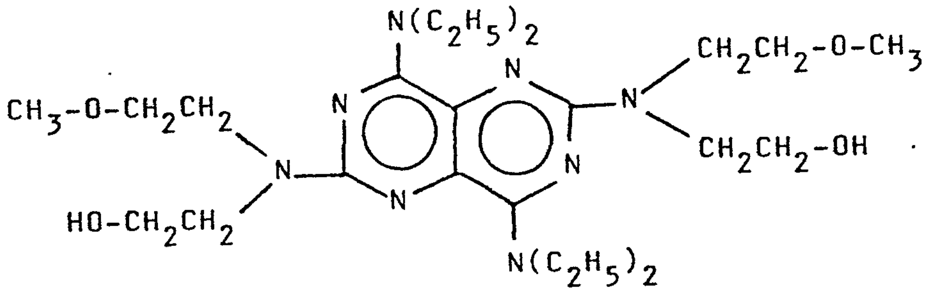

(B)

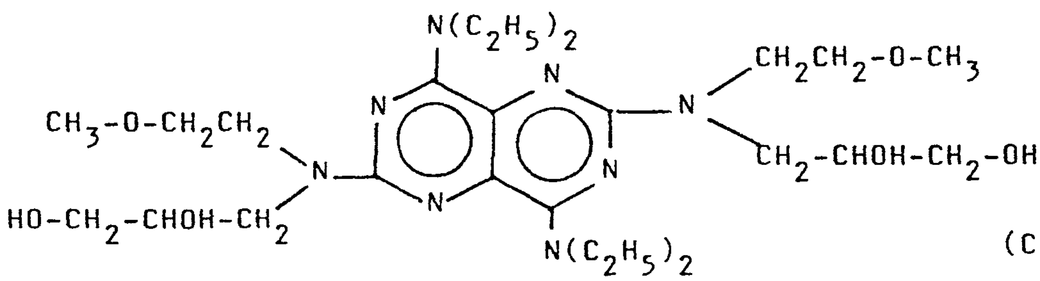

(C)

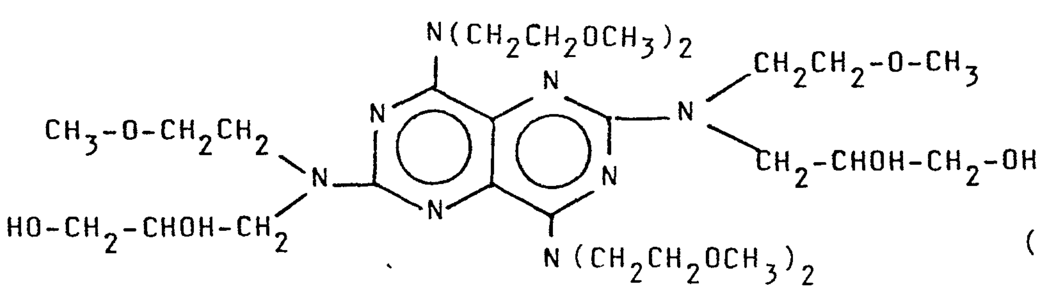

(D)

sowie 2,6-Bis-(2'-hydroxyethyl-2"-methoxyethyl-amino)-4,8-dipyrrolidino-pyrimido[5,4-d]pyrimidin (E)
und die entsprechende

4,8-Dipiperidino-Verbindung (F)

erwiesen.

Für die therapeutische Anwendung werden die Verbindungen der Formel I mit gebräuchlichen Hilfs- und/oder Trägerstoffen zu üblichen galenischen Zubereitungen verarbeitet, beispielsweise zu Tabletten, Dragées, Suppositorien, Ampullen, Lösungen, Kapseln.

Tabletten (Angaben in Gewichtsteilen)

10 Teile 4,8-Bis-(diethylamino)-2,6-bis-(2'-hydroxyethyl-2"-methoxyethylamino)-pyrimido[5,4-d] Pyrimidin

10 Teile kolloidale Kieselsäure

110 Teile Milchzucker

58 Teile Maisstärke

6 Teile Polyvinylpyrrolidon

4 Teile Natriumcelluloseglykolat

2 Teile Magnesiumstearat

Die Bestandteile werden in üblicher Weise zu Tabletten von 200 mg verarbeitet.

Kapseln

1 Kapsel enthält

60 mg Wirkstoff nach Formel I

170 mg Laktose

170 mg Maisstärke

Patentansprüche

1. Verwendung von Verbindungen der Formel

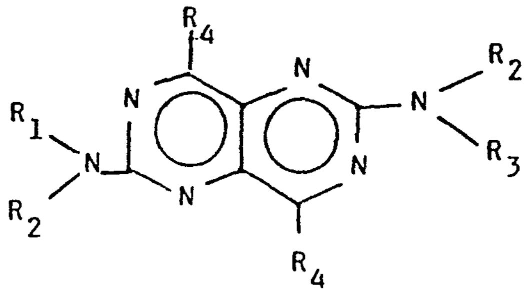

(I)

in der

$R_1$ einen $C_1$-$C_3$-Alkoxy-($C_2$-$C_3$-alkyl)rest,

$R_2$ einen $C_2$-$C_6$-Hydroxyalkylrest oder einen $C_3$-Dihydroxyalkylrest,

$R_3$ einen $C_1$-$C_3$-Alkoxy-($C_2$-$C_3$-alkyl)rest oder einen $C_2$-$C_3$-Hydroxyalkylrest,

$R_4$ einen Dialkylaminorest, wobei jeder Alkylrest 1 bis 6 C-Atome enthalten kann, die Diallyl- oder Di-(methoxyethyl)-aminogruppe oder einen gegebenenfalls durch eine Methylgruppe substituierten Pyrrolidino-, Piperidino-, Hexamethylenamino-, 1', 2', 5', 6'- Tetrahydropyridino-, Morpholino-, Thiomorpholino- oder 1-Oxidothiomorpholinoring, als freie Basen oder in Form von Säureadditionssalzen mit anorganischen oder organischen Säuren bei der Herstellung von Arzneimitteln zur Behandlung der zerebralen Insuffizienz.

2. Verwendung von Verbindungen der Formel I bei der Herstellung von Arzneimitteln zur Erhöhung der cerebralen Leistungsfähigkeit.

3. Verwendung von Verbindungen der Formel I bei der Herstellung von Arzneimitteln zur Behandlung der cerebralen Insuffizienz.

4. Verwendung nach Anspruch 1 bis 3, dadurch gekennzeichnet, daß als Verbindung der Formel I 4,8-Bis-(diethylamino)-2,6-bis-(2'-hydroxyethyl-2"-methoxyethylamino)-pyrimido[5,4-d]pyrimidin und seine physiologisch verträglichen Säureadditionssalze dienen.

5. Verwendung nach Anspruch 1 bis 3, dadurch gekennzeichnet, daß als Verbindung der Formel I 4,8-Bis-(ethyl-n-butylamino)-2,6-bis-(2'-hydroxyethyl-2"-methoxyethylamino)-pyrimido[5,4-d]pyrimidin und seine physiologisch verträglichen Säureadditionssalze dienen.

6. Verwendung nach Anspruch 1 bis 3, dadurch gekennzeichnet, daß als Verbindung der Formel I 4,8-Bis-(di-(2-methoxyethyl)amino)-2,6-bis-(2'-hydroxyethyl-2"-methoxyethylamino)-pyrimido[5,4-d]pyrimidin und seine physiologisch verträglichen Säureadditionssalze dienen.

7. Verwendung nach Anspruch 1 bis 3, dadurch gekennzeichnet, daß die als Wirkstoff, gegebenenfalls in Form ihrer Säureadditionssalze benutzten Verbindungen der Formel

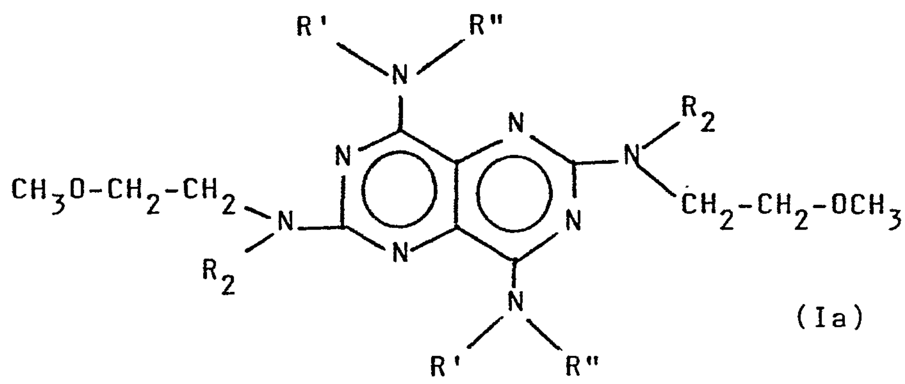

(Ia)

entsprechen,
worin $R_2$ die obige Bedeutung hat und R' einen gegebenenfalls durch Sauerstoff unterbrochenen $C_2$-$C_4$-Alkylrest, R" die Ethyl-, 2-Methoxyethyl- oder Allylgruppe darstellt.

8. Verwendung nach Anspruch 7 , dadurch gekennzeichnet, daß $R_2$ in der Formel Ia 2-Hydroxyethyl ist.

9. Verwendung nach Anspruch 1 bis 3, dadurch gekennzeichnet, daß als Verbindung der Formel I 2,6-Bis-(2'-hydroxyethyl-2"-methoxyethylamino)-4,8-dipyrrolidino-pyrimido[5,4-d]pyrimidin oder seine physiologisch annehmbaren Säureadditionssalze dienen.

1o. Verwendung nach Anspruch 1 bis 3, dadurch gekennzeichnet, daß als Verbindung der Formel I 2,6-Bis-(2'-hydroxyethyl-2"-methoxyethylamino)-4,8-dipiperidino-pyrimido[5,4-d]pyrimidin oder seine physiologisch annehmbaren Säureadditionssalze dienen.